# EUROPEAN PATENT SPECIFICATION

(11) **EP 1 375 087 B1**
(45) Date of publication and mention of the grant of the patent: **10.06.2009**
(21) Application number: 02715842.7
(22) Date of filing: 21.01.2002
(51) Int. Cl.: B25J 15/08

(54) **MULTI-FINGER HAND DEVICE**
HANDVORRICHTUNG MIT MEHREREN FINGERN
DISPOSITIF DE MAIN A PLUSIEURS DOIGTS

(30) Priority: 23.01.2001 JP 2001014292
(43) Date of publication of application: 02.01.2004
(73) Proprietor: HONDA GIKEN KOGYO KABUSHIKI KAISHA, Minato-ku, Tokyo 107-8556 (JP)
(72) Inventor: MATSUDA, Hiroshi, KK HONDA GIJYUTSU KENKYUSHO, Wako-shi, Saitama 351-0193 (JP)
(74) Representative: Hague, Alison Jane
(86) International application number: PCT/JP2002/000375
(87) International publication number: WO 2002/058896

(56) References cited:
- JP-A- 8 132 381
- JP-A- 8 323 674
- JP-A- 8 323 676
- JP-A- 59 209 785
- JP-U- 61 187 690
- US-A- 5 062 855

## Description

### Technical field:

The present invention relates to a multi-fingered hand device for robots.

More specifically, the invention relates to a multi-fingered hand device according to the preamble of claim 1.

Such a device is known from JP-A-61 187 690.

### Background art:

There have heretofore been known multi-fingered hand devices for robots such as human-shaped robots, the multi-fingered hand devices having a plurality of finger mechanisms, which correspond to human fingers, extending from a palm assembly. Each of the finger mechanisms comprises a plurality of interjoint members, which correspond to phalanxes of human fingers, connected by joints. The finger mechanisms can be bent and extended when the interjoint members are turned about the joints.

The interjoint members of the finger mechanisms are usually made of a hard material such as metal or the like. Therefore, when an object is gripped by a plurality of finger mechanisms of a multi-fingered hand device, the object may be damaged by the finger mechanisms, and when the multi-fingered hand device is moved, a finger mechanism thereof may hit the object, possibly damaging the object.

The present invention has been made in view of the above background. It is an object of the present invention to provide a multi-fingered hand device which is capable of gripping an object without causing damage to the object, is prevented from being smeared, and has increased maintainability.

JP-A-61187690 discloses a multi-fingered hand device in which the fingers are provided with damping members.

JP-A-8132381 discloses a hand device in which tubular resilient cover members are fitted over the finger mechanisms.

### Disclosure of the invention:

To achieve the above object, there is provided a multi-fingered hand device as defined in claim 1.

With the present invention, since the damping members are mounted on the respective interjoint members of the finger mechanisms in covering relation to outer peripheral surfaces of the interjoint members, shocks produced when the finger mechanisms hit an object are lessened. As the damping members on adjacent ones of the interjoint members of the finger mechanisms are spaced from each other at the joint disposed between the adjacent interjoint members, the finger mechanisms can be bent at the joints without fail.

Therefore, the multi-fingered hand device according to the present invention is capable of gripping an object without causing damage to the object.

The damping members may be made of urethane rubber of the like, for example.

If the damping members of the finger mechanisms are exposed, then when the multi-fingered hand device grips an object, the damping members of the finger mechanisms are brought into direct contact with the object, and hence tend to be smeared. The damping members are liable to be relatively expensive because they are generally required to have a certain thickness for a desired damping capability.

The resilient cover members fitted over the finger mechanisms according to the present invention prevent the damping members from being unduly smeared. Since the resilient cover members may be relatively thin, they may be relatively inexpensive. As the resilient cover members are extensible and contractible, the finger mechanisms can be bent without fail.

The resilient cover members of the respective finger mechanisms are shaped complementarily to outer profiles of the finger mechanisms as they are extended such that a restoring force is produced to bias the finger mechanisms to an extended position when the finger mechanisms are bent.

Specifically, in multi-fingered hand devices, the finger mechanisms are generally biased to an extended position by biasing means such as springs, and are bent by drive power from an actuator such as an electric motor or the like. However, in the case where the resilient cover members of the respective finger mechanisms are shaped complementarily to outer profiles of the finger mechanisms as they are extended, when the finger mechanisms are bent, the finger mechanisms are biased to the extended position under a restoring force of the resilient cover members. As a result, biasing forces of the biasing means may be reduced.

According to the present invention, furthermore, the front walls of the damping members have such a thickness for preventing the curved portions from being pressed against the joints. Therefore, the curved portions are prevented from being caught by the joints and hence damaged thereby, and hence the joints are prevented from suffering a malfunction which would otherwise occur if the curved portions were caught by the joints.

In the multi-fingered hand device according to the present invention which has the resilient cover members fitted over the finger mechanisms, the finger mechanisms can be bent and extended by turning the inter-joint members about the joints, and the resilient cover members of the respective finger mechanisms are arranged to cause front walls thereof to be curved at the joints of the finger mechanisms toward the joints when the finger mechanisms are bent.

With the above arrangement, when the finger mechanisms are bent, since the front walls of the resilient cover members of the finger mechanisms are curved so as to be concave inwardly at the joints of the finger mechanisms toward the joints, the front walls of the resilient cover members do not project outwardly (in a direction away from the joints) at the bent regions of the finger mechanisms. Consequently, when the multi-fingered hand device is operating, the resilient cover members do not interfere with the operation of the multi-fingered hand device, and are not caught by foreign matter.

In order to allow the front walls of the resilient cover members to be curved at the joints of the finger mechanisms toward the joints, recesses are defined in the portions of the front walls of the resilient cover members at the joints.

The recesses are preferably spread toward the front walls of the resilient cover members.

with the above arrangement, when the finger mechanisms are bent, the front walls of the resilient covers are bent at the joints, more reliably toward the joints. As a result, when the finger mechanisms are bent, the front walls of the resilient covers are prevented from partly projecting outwardly into interference with the operation of the hand device.

Preferably, the resilient cover members of the respective finger mechanisms are thicker in at least a portion of back walls thereof behind the finger mechanisms than in front walls thereof.

When the finger mechanisms are bent, the restoring force of the resilient cover members that are bent together with the finger mechanism is increased, making it possible to further reduce the biasing forces of the biasing means such as springs or the like for biasing the finger mechanisms to the extended position.

According to the present invention, furthermore, it is preferable for the multi-fingered hand device to have a resilient cover member integrally joined to at least one of the resilient cover members of the respective finger mechanisms and removably mounted on the palm assembly in covering relation to a surface of the palm assembly.

With the above arrangement, the palm assembly together with the finger mechanism is covered with the resilient cover members to protect the hand device in its entirety against being smeared. Because the resilient cover member of the palm assembly is integrally joined to at least one of the resilient cover members of the respective finger mechanisms, the number of parts of the resilient cover members can be reduced.

According to the present invention, moreover, the resilient cover members of the respective finger mechanisms and the resilient cover member of the palm assembly should preferably be made of an elastomer. The resilient cover members can thus be manufactured relatively inexpensively.

Gaps between the damping members on adjacent ones of the interjoint members of the finger mechanisms should preferably be shaped as to be spread from the joint between the adjacent interjoint members toward the front surface of the finger mechanisms. With this structure, exposed portions of the joints of the finger mechanisms which are not covered with the damping members may be minimized without obstructing the bending action of the finger mechanisms at the joints.

### Brief description of the drawings:

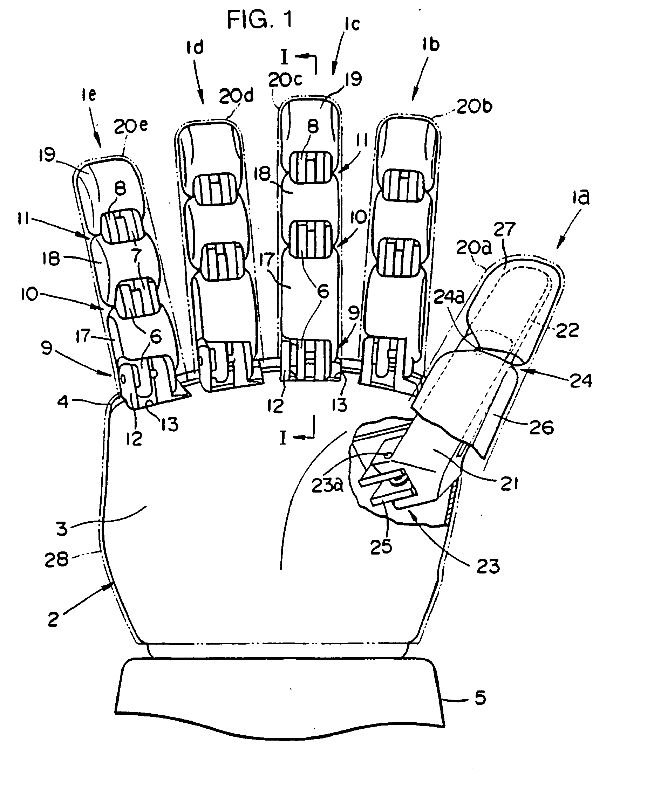

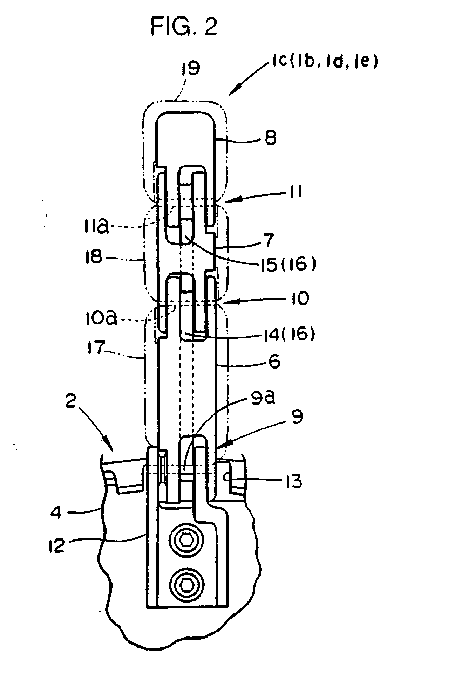

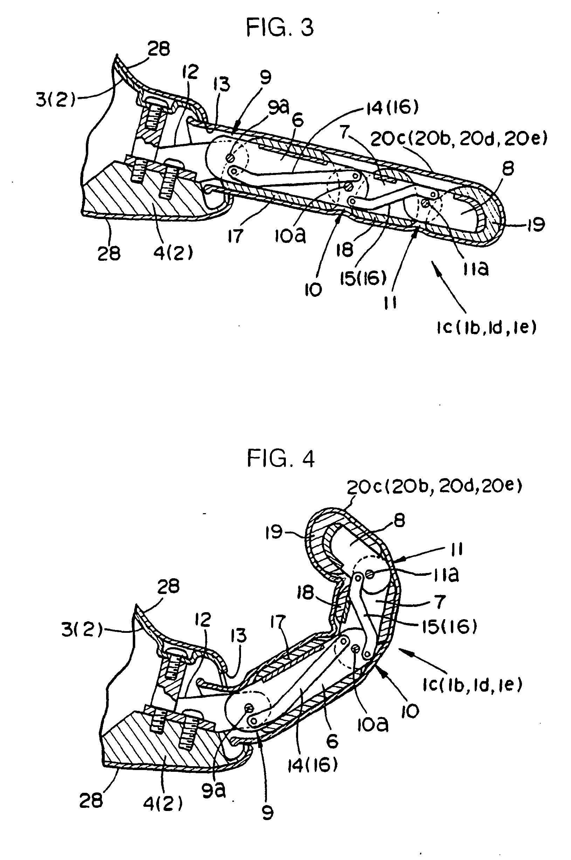

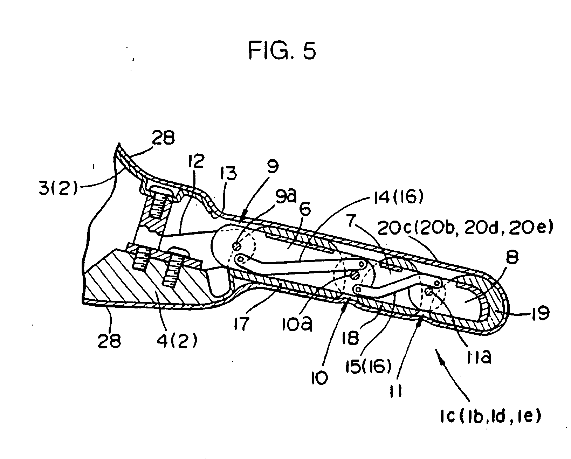

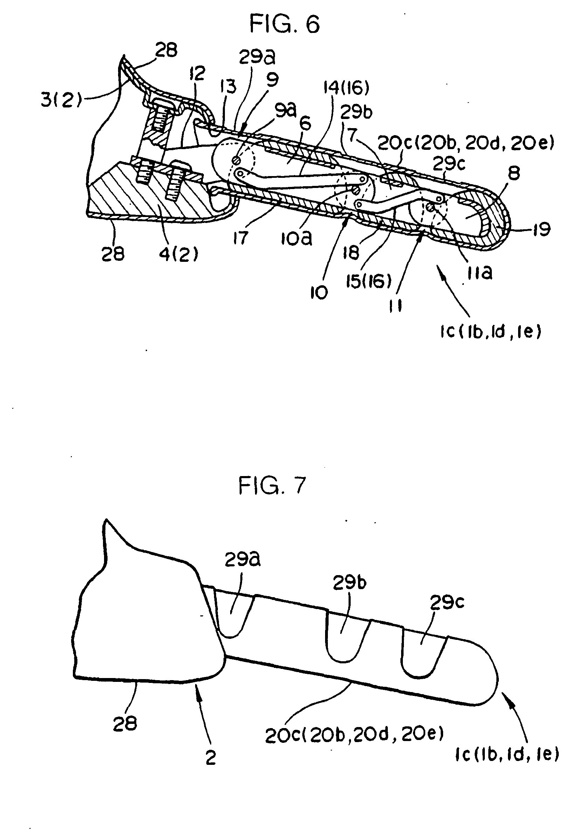

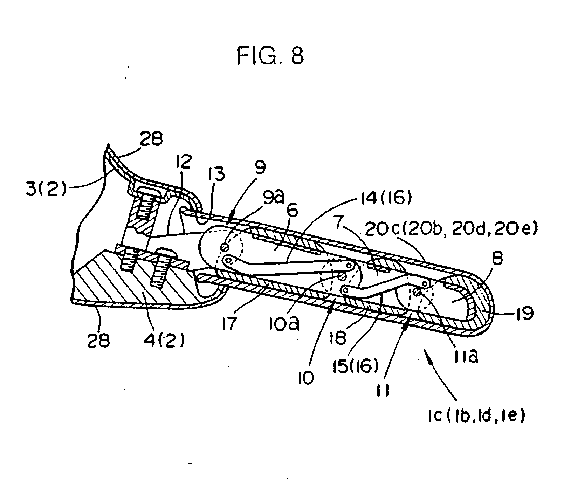

FIG. 1 is a plan view of a multi-fingered hand device, for explanatory purposes, showing a multi-fingered hand device from its palm side; FIG. 2 is a plan view showing essential parts of the multi-fingered hand device shown in FIG. 1; FIG. 3 is a cross-sectional view taken along line I - I of FIG. 1; FIG. 4 is a view illustrative of the manner in which a finger mechanism of the multi-fingered hand device shown in FIG. 1 operates; FIG. 5 is a longitudinal cross-sectional view of a finger mechanism of another multi-fingered hand device illustrated for explanatory purposes; FIG. 6 is a longitudinal cross-sectional view of a finger mechanism of a multi-fingered hand device according to an embodiment of the present invention; FIG. 7 is a side elevational view of the finger mechanism shown in FIG. 6; and FIG. 8 is a longitudinal cross-sectional view of a finger mechanism of another multi-fingered hand device illustrated for explanatory purposes.

### Best mode for carrying out the invention:

A multi-fingered hand device will be described below for explanatory purposes with reference to FIGS. 1 through 4.

As shown in FIG. 1, a first illustrated multi-fingered hand device 1 has a structure similar to human hands and comprises five finger mechanisms 1a - 1e extending from a palm assembly 2. The finger mechanisms 1a - 1e correspond respectively to the thumb, index finger, middle finger, ring finger and little finger of a human hand.

The palm assembly 2 comprises an assembly of a hand palm forming member 3 and a hand back forming member 4 for forming the face of a hand palm (face side in FIG. 1) and the face of a hand back (reverse side in FIG. 1), respectively. A space defined between the hand palm forming member 3 and the hand back forming member 4 (an internal space in the palm assembly 3) serves as a region for accommodating therein drive mechanisms (not shown) for operating the finger mechanisms 1a - 1e. The palm assembly 2 has a proximal end connected to an arm 5 of a robot.

Of the finger mechanisms 1a - 1e, the finger mechanisms 1b - 1e other than the finger mechanism la which corresponds to the thumb extend substantially forward (upward in FIG. 1) from a distal end of the palm assembly 2 (an end of the palm assembly 2 which is opposite to the proximal end connected to the arm 5). These four finger mechanisms 1b - 1e are identical in basic structure to each other. The structure of these four finger mechanisms 1b **-** 1e will be described below.

FIGS. 2 through 4 are views showing the structure of the finger mechanism 1c which corresponds to the middle finger. However, since the finger mechanisms 1b-1e are identical in basic structure to each other, but are different in partial size from each other, FIGS. 2 through 4 will be referred to as representing the structure of each of the finger mechanisms 1b - 1e.

As shown in FIGS. 2 and 3, each of the finger mechanisms 1b **-** 1e has three interjoint members 6 - 8 and three joints 9 - 11, and is of such a structure that the interjoint members 6 - 8 are successively joined by the joints 9 - 11 from a fixed member 12 that is fixed to the palm assembly 2. The fixed member 12 is fixed to the hand back forming member 4 within the palm assembly 2, as shown in FIG. 3, and has an end projecting through an opening 13 that is defined in the distal end of the palm assembly 2 in alignment with each of the finger mechanisms 1b - 1e. The interjoint member 6 is connected to an end of the fixed member 12 by the joint 9.

In FIG. 1, the above components of only the finger mechanisms 1c, 1e are denoted by reference characters, and reference characters of the components of the finger mechanisms 1b, 1d are omitted from illustration.

As shown in FIGS. 2 and 3, the joints 9 - 11 have respective joint shafts 9a, 10a, 11a as rotational shafts oriented in the same direction (substantially in the transverse direction of the palm assembly 2). The interjoint members 6 - 8 can be turned respectively about the axes of the joint shafts 9a - 11a with respect to the fixed member 12, the interjoint member 6, and the interjoint member 7, respectively. The finger mechanisms 1b-1e can be bent and extended by turning the interjoint members 6 - 8 respectively about the joints 9 - 11.

In the first illustrated multi-fingered hand device, the finger mechanisms 1b **-** 1e have respective link mechanisms 16 each comprising a link arm 14 coupling the fixed member 12 and the interjoint member 7 to each other, and a link arm 15 coupling the interjoint member 6 and the interjoint member 8 to each other. The link arm 14 has an end rotatably supported on the fixed member 12 at a position which is closer to a back surface of the finger mechanisms 1b - 1e (a lower surface in FIG. 3) than the joint shaft 9a, and another end rotatably supported on the interjoint member 7 at a position which is closer to a front surface of the finger mechanisms 1b - 1e (an upper surface in FIG. 3) than the joint shaft 10a. The link arm 15 has an end rotatably supported on the interjoint member 6 at a position which is closer to the back surface of the finger mechanisms 1b - 1e than the joint shaft 10a, and another end rotatably supported on the interjoint member 8 at a position which is closer to the front surface of the finger mechanisms 1b **-** 1e than the joint shaft 11a.

Because of the link mechanism 16 constructed of the link arms 14, 15, when the interjoint member 6 is turned with respect to the fixed member 12, the interjoint member 7 is turned with respect to the interjoint member 6, and the interjoint member 8 is turned with respect to the interjoint member 7, in ganged relation to the interjoint member 7. Each of the finger mechanisms 1b **-** 1e is thus bent and extended by turning the interjoint member 6 about the joint shaft 9a with respect to the fixed member 12.

Although not shown as they do not represent essential part of the present invention, the interjoint member 6 of each of the finger mechanisms 1b - 1e is biased to an extended position (shown in FIG. 3) by a spring (biasing means), with the result that the finger mechanisms 1b - 1e are biased in their entirety to an extended position. When a drive mechanism comprising a wire, pulleys, an electric motor, etc. imparts rotational drive power to the interjoint member 6 against the bias of the spring, the finger mechanisms 1b - 1e are bent, i.e., the interjoint members 6 - 8 are turned toward the palm side of the palm assembly 2, as shown in FIG. 4.

As shown in FIGS. 1 through 3, damping members 17 **-** 19 are mounted respectively on the interjoint members 6 - 8 of the finger mechanisms 1b - 1e in covering relation to outer peripheral surfaces thereof. The damping members 17 - 19 are made of urethane rubber or the like, for example.

As shown in FIG. 3, of the damping members 17 - 19, the damping member 17 mounted on the interjoint member 6 and the damping member 18 mounted on the interjoint member 7 next to the damping member 17 are spaced from each other at the joint 10 between the interjoint members 6, 7. Similarly, the damping member 18 mounted on the interjoint member 7 and the damping member 19 mounted on the interjoint member 8 are spaced from each other at the joint 11 between the interjoint members 7, 8. When the finger mechanisms 1b - 1e are bent from an extended position, the damping members 17, 18 that are positioned next to each other are brought toward each other at a position which is closer to the front surface of the finger mechanisms 1b - 1e than the joint shaft 10a and are spaced away from each other at a position which is closer to the back surface of the finger mechanisms 1b - 1e than the joint shaft 10a. Accordingly, the gap between the damping members 17, 18 is shaped so as to spread toward the front surface of the finger mechanisms 1b **-** 1e such that the damping members 17, 18 will not abut against and interfere with each other at the position closer to the front surface of the finger mechanisms 1b - 1e than the joint shaft 10a when the finger mechanisms 1b - 1e are bent (see FIG. 3). The gap between the damping members 18, 19 is similarly shaped so as to spread toward the front surface of the finger mechanisms 1b - 1e at the position closer to the front surface of the finger mechanisms 1b - 1e than the joint shaft 11a (see FIG. 3).

Resilient finger cover members 20b **-** 20e are removably fitted respectively over the finger mechanisms 1b **-** 1e in covering relation to the outer surfaces of the finger mechanisms 1b - 1e including the damping members 17 **-** 19. The resilient finger cover members 20b - 20e comprise thin tubular members having closed ends at the tip ends of the finger mechanisms 1b **-** 1e. The resilient finger cover members 20b - 20e are shaped complementarily to the outer profiles of the corresponding finger mechanisms 1b - 1e as they are extended (the diameters of the resilient finger cover members 20b - 20e are substantially equal to the outside diameters of the damping members 17 - 19 of the finger mechanisms 1b - 1e). As shown in FIG. 3, the other end (open end) of each of the resilient finger cover members 20b - 20e is inserted into the palm assembly 2 through the opening 13 therein. The resilient finger cover members 20b - 20e are made of an elastomer such as rubber or the like and are extensible and contractible.

As shown in FIG. 1, the finger mechanism 1a corresponding to the thumb extends obliquely forward from a side (rearward of the finger mechanism 1b) of the palm assembly 2, and has two interjoint members 21, 22 and two joints 23, 24 as its major members. The finger mechanism 1a is such a structure that the interjoint members 21, 22 are successively joined by the joints 23, 24 from the fixed member 25 that is fixed to the hand back forming member 4 within the palm assembly 2.

The joints 23, 24 have respective joint shafts 23a, 24a as rotational shafts. The interjoint members 21, 22 can be turned respectively about the axes of the joint shafts 23a, 24a with respect to the fixed member 25 and the interjoint member 21, respectively. The finger mechanism 1a can be bent and extended by turning the interjoint members 21, 22 respectively about the joints 23, 24.

Although not shown, the interjoint members 21, 22 of the finger mechanism 1a are biased to an extended position (shown in FIG. 1) by a spring (biasing means). When a drive mechanism comprising a wire, pulleys, an electric motor, etc. imparts rotational drive power to the interjoint member 22 against the bias of the spring, the finger mechanism 1a is bent, i.e., the interjoint members 21, 22 are turned to bring the tip end of the finger mechanism 1a toward the finger mechanisms 1b - 1e when the finger mechanisms 1b - 1e are bent.

As with the finger mechanisms 1b - 1e, damping members 26, 27 are mounted respectively on the interjoint members 21, 22 of the finger mechanism 1a in covering relation to outer peripheral surfaces thereof. The damping members 26, 27 are spaced from each other at the joint 24 therebetween. As with the finger mechanisms 1b - 1e, the gap between the damping members 26, 27 is shaped so, as to spread toward the front surface of the finger mechanism 1a at the position closer to the front surface of the finger mechanism 1a than the joint shaft 24a.

Furthermore, as with the finger mechanisms 1b - 1e, a resilient finger cover member 20a in the form of a thin tubular member is removably fitted over the finger mechanism 1a in covering relation to the outer surface of the finger mechanism 1a including the damping members 27, 28, the resilient finger cover member 20a having a closed end at the tip end of the finger mechanism 1a. As with the resilient finger cover members 20b - 20e for the finger mechanisms 1b - 1e, the resilient finger cover member 20a is made of an elastomer such as rubber or the like and can be stretched and contracted. The resilient finger cover member 20a is shaped complementarily to the outer profile of the finger mechanism 1a as it is extended (the diameter of the resilient finger cover member 20a is substantially equal to the outside diameters of the damping members 26, 27 of the finger mechanism 1a).

In the present arrangement, the finger cover members 20a - 20e have a substantially uniform thickness.

A thin palm cover member 28 (see the imaginary lines in FIG. 1 and also see FIGS. 3 and 4) is removably mounted on the palm assembly 2 so as to cover the overall outer peripheral surface of the palm assembly 2 except for the regions where the finger mechanisms 1a **-** 1e extend from the palm assembly 2. The palm cover member 28 is made of an elastomer as with the finger cover members 20a - 20e.

With the multi-fingered hand device according to the illustrated arrangement, since the damping members 17 - 19, 26, 27 are mounted on the interjoint members 6 - 8, 21, 22 of the finger mechanisms 1a **-** 1e, even if the finger mechanisms 1a - 1e abut against an object when the multi-fingered hand device grips the object, no excessive shocks are applied to the object, thus avoiding undue damage to the object. Adjacent ones of the damping members 17 - 19, 26, 27 of the finger mechanisms 1a - 1e are spaced from each other at the joint disposed therebetween. In particular, the gap between adjacent ones of the damping members 17 - 19, 26, 27 is shaped so as to spread toward the front surface of the finger mechanisms 1a **-**1e at the position closer to the front surface than the joint shaft of the joint. Therefore, when the finger mechanisms 1a - 1e are bent, the damping members are prevented from abutting against each other, allowing the finger mechanisms 1a - 1e to be bent without fail.

Inasmuch as the finger mechanisms 1a - 1e including the damping members 17 - 19, 26, 27 are covered with the resilient finger cover members 20a - 20e, the major members of the finger mechanisms 1a - 1e and the damping members 17 - 19, 26, 27 are prevented from being smeared and worn. Particularly, the damping members 17-19, 26, 27, which are likely to be relatively thick and expensive for a desired damping capability, can have their service life increased by preventing themselves from smear and wear. A lubricant such as grease or the like is often applied to the joints 9 - 11, 23, 24 of the finger mechanisms 1a - 1e. Since the finger mechanisms 1a - 1e are covered with the resilient finger cover members 20a - 20e, the lubricant is prevented from being attached to the surfaces of the multi-fingered hand device and scattered around.

The resilient finger cover members 20a - 20e and the palm cover member 28 are smeared when the multi-fingered hand device is in operation. Since these cover members are thin and can be manufactured relatively inexpensively, they can be replaced at low expenses. The resilient cover members 20a - 20e, 28 can be removed from the multi-fingered hand device and cleaned.

When the finger mechanisms 1b - 1e are bent, the front surfaces of the resilient finger cover members 20b - 20e on the finger mechanisms 1b - 1e are basically curved toward the joints 9 - 11 at the respective joints 9 **-** 11, as shown in FIG. 4. At this time, the curved portions enter into the gaps at the joints 10, 11 on the front surface of the adjacent damping members (17, 18), (18,19). In the illustrated arrangement, the thickness of the damping members 17 - 19 on their front surface is of such a value that the curved portions of the resilient finger cover members 20b - 20e are not pressed against the joints 9 **-** 11. Therefore, those curved portions are prevented from being caught and damaged by the joints 9-11, and hence the joints 9 **-** 11 are prevented from suffering a malfunction which would otherwise occur if the curved portions were caught by the joints 9 - 11. Though not illustrated, this holds true also for the resilient finger cover member 20a of the finger mechanism 1a corresponding to the thumb.

The resilient finger cover members 20a - 20e of the finger mechanisms 1a - 1e comprise resilient members shaped complementarily to the outer profiles of the corresponding finger mechanisms 1a - 1e as they are extended. When the finger mechanisms 1a - 1e are bent, the resilient finger cover members 20a - 20e produce a restoring force for biasing the finger mechanisms 1a - 1e to an extended position. Therefore, the springs (not shown) used to bias the finger mechanisms 1a - 1e to an extended position may have their biasing forces reduced, and hence may be reduced in weight and size.

A second multi-fingered hand device will be described below for explanatory purposes with reference to FIG. 5. The second multi-fingered hand device has a basic structure that is identical to the basic structure of the first multi-fingered hand device described with reference to FIGS. 1 to 4, and differs from the first multi-fingered hand device as to only partial structural details of resilient finger cover members and a resilient palm cover member. Therefore, those parts of the multi-fingered hand device according to the second arrangement which are identical to those of the multi-fingered hand device according to the first arrangement are denoted by identical reference characters, and will not be described in detail below.

FIG. 5, which is similar to FIG. 3, shows structural details of the finger mechanisms 1b - 1e other than the finger mechanism 1a which corresponds to the thumb. As shown in FIG. 5, each of the resilient finger cover members 20b - 20e fitted over the respective finger mechanisms 1b - 1e has an end, near the palm assembly 2, which is integrally joined to the resilient palm cover member 28. Although not shown, the resilient finger cover member 20a fitted over the finger mechanism 1a corresponding to the thumb similarly has an end, near the palm assembly 2, which is integrally joined to the resilient palm cover member 28. According to the second illustrated arrangement, therefore, the resilient finger cover members 20a - 20e and the palm cover member 28 are integrally formed in the shape of a glove. Other structural details are exactly identical to those of the multi-fingered hand device according to the first arrangement.

The multi-fingered hand device according to the second arrangement operates in the same manner, and offers the same advantages, as the multi-fingered hand device according to the first arrangement. In addition, since the resilient finger cover members 20a - 20e and the palm cover member 28 which are mounted on the multi-fingered hand device are unitary, the number of parts of those cover members is smaller than with the multi-fingered hand device according to the first arrangement, and the inventory control for those parts is facilitated.

In the second arrangement, all the resilient finger cover members 20a - 20e are integrally formed with the palm cover member 28. However, only some (one through four) of the resilient finger cover members 20a-20e may be integrally formed with the palm cover member 28 in view of the ease with which those cover members are mounted on the multi-fingered hand device.

An embodiment of the present invention will be described below with reference to FIGS. 6 and 7. A multi-fingered hand device according to the illustrated embodiment has a basic structure that is identical to the basic structure of the first illustrated multi-fingered hand device, and differs from the first arrangement of multi-fingered hand device as to only partial structural details of resilient finger cover members. Therefore, those parts of the multi-fingered hand device according to the illustrated embodiment which are identical to those of the multi-fingered hand device according to the first arrangement illustrated for explanatory purposes are denoted by identical reference characters, and will not be described in detail below.

FIG. 6, which is similar to FIG. 3, shows structural details of the finger mechanisms 1b - 1e other than the finger mechanism 1a which corresponds to the thumb. As shown in FIG. 6, each of the resilient finger cover members 20b - 20e fitted over the respective finger mechanisms 1b - 1e has recesses 29a, 29b, 29c defined in the front wall thereof at the respective joints 9 - 11. As shown in FIG. 7, these recesses 29a, 29b, 29c extend from the front wall toward side walls of the resilient finger cover members 20b - 20e. As shown in FIG. 6, the thickness of the portions of the resilient finger cover members 20b - 20e which are positioned at the recesses 29a, 29b, 29c is smaller than the thickness of the other portions of the resilient finger cover members 20b - 20e. Though not illustrated, this holds true also for the resilient finger cover member 20a fitted over the finger mechanism 1a corresponding to the thumb. The resilient finger cover member 20a has recesses defined therein at the joints 23, 24. Other structural details are exactly identical to those of the multi-fingered hand device according to the first illustrated arrangement.

The multi-fingered hand device according to the present embodiment operates in the same manner, and offers the same advantages, as the multi-fingered hand device according to the first arrangement and is further advantageous as follows:

According to the first arrangement, since the front wall of each of the resilient finger cover members 20a - 20e of the finger mechanisms 1a - 1e is uniformly thick and smooth (see FIG. 3), when the finger mechanisms 1a - 1e are bent, the front wall of the resilient finger cover members 20a **-** 20e at the joints 9 - 11 may be curved so as to project outwardly depending on how much the resilient finger cover members 20a - 20e are deteriorated.

According to the embodiment of the invention, as shown in FIG. 7, the front wall of each of the resilient finger cover members 20b - 20e of the finger mechanisms 1b - 1e has the recesses 29a - 29c positioned at the respective joints 9 - 11, and the thickness of the portions of the resilient finger cover members 20b - 20e which are positioned at the recesses 29a - 29c is smaller than the thickness of the other portions of the resilient finger cover members 20b - 20e.

Therefore, when the finger mechanisms 1b - 1e are bent, the front wall of each of the resilient finger cover members 20b'- 20e is curved more reliably toward the joints 9 - 11 at the respective joints 9 - 11. Specifically, when the finger mechanisms 1b - 1e are bent to the shape shown in FIG. 4, the front wall of each of the resilient finger cover members 20b - 20e is curved at the respective joints 9 - 11, and is curved more reliably in the shape shown in FIG. 4. This also holds true for the resilient finger cover member 20a of the finger mechanism 1a corresponding to the thumb.

As a result, with the multi-fingered hand device according to the present embodiment, when the finger mechanisms 1a - 1e are bent, the front wall of each of the resilient finger cover members 20a - 20e is prevented from partly projecting outwardly into interference with the operation of the hand device.

All or some of the resilient finger cover members 20a - 20e according to the present embodiment may be integrally joined to the palm cover member 28 as with the multi-fingered hand device according to the second explanatory arrangement.

Another multi-fingered hand device illustrated for explanatory purposes will be described below with reference to FIG. 8. A multi-fingered hand device according to this arrangement has a basic structure that is identical to the basic structure of the multi-fingered hand device according to the first arrangement, and differs from the multi-fingered hand device according to the first arrangement as to only partial structural details of resilient finger cover members. Therefore, those parts of the multi-fingered hand device according to the arrangement illustrated with respect to FIG. 8 which are identical to those of the multi-fingered hand device according to the first explanatory arrangement are denoted by identical reference characters, and will not be described in detail below.

FIG. 8, which is similar to FIG. 3, shows structural details of the finger mechanisms 1b - 1e other than the finger mechanism 1a which corresponds to the thumb. As shown in FIG. 8, each of the resilient finger cover members 20b - 20e fitted over the respective finger mechanisms 1b - 1e has a back wall thicker than a front wall thereof. Though not illustrated, this holds true also for the resilient finger cover member 20a fitted over the finger mechanism 1a corresponding to the thumb, i.e., resilient finger cover member 20a has a back wall thicker than a front wall thereof. Other structural details are exactly identical to those of the multi-fingered hand device according to the first arrangement.

The multi-fingered hand device according to the arrangement of FIG. 8 operates in the same manner, and offers the same advantages, as the multi-fingered hand device according to the first arrangement. In addition, because each of the resilient finger cover members 20a-20e fitted over the respective finger mechanisms 1a **-** 1e is thicker in the back wall than in the front wall, when the finger mechanisms 1a - 1e are bent, the resilient finger cover members 20a - 20e undergo greater forces tending to restore them to an extended position than with the multi-fingered hand device according to the first arrangement. As a consequence, the springs (not shown) used to bias the finger mechanisms 1a - 1e to an extended position may have their biasing forces reduced, and hence may be reduced in weight and size.

All or some of the resilient finger cover members 20a - 20e according to the arrangement of FIG. 8 may be integrally joined to the palm cover member 28 as with the multi-fingered hand device according to the second illustrated arrangement.

### Industrial applicability:

As described above, the multi-fingered hand device according to the present invention is useful as a multi-fingered hand device of a robot for performing various operations with a plurality of finger mechanisms as they are bent and extended.

## Claims

1. A multi-fingered hand device having a plurality of finger mechanisms (1a-1e) extending from a palm assembly (2), each comprising:
a plurality of inter-joint members (6,7,8,21,22) successively connected by a plurality of finger joints (9,10,11,23,24), wherein said finger mechanisms (1a-1e) can be bent and extended when said interjoint members (6,7,8,21,22) are turned about the joints (9,10,11,23,24);
and damping members (17,18,19,26,27) mounted on the respective interjoint members (6,7,8,21,22) of the finger mechanisms in covering relation to outer peripheral surfaces of the interjoint members, the damping members (17,18,19,26,27) being mounted respectively on adjacent ones of the interjoint members (6,7,8,21,22) of the finger mechanisms (1a-1e) and being spaced from each other at the joint (9,10,11,23,24) disposed between the adjacent ones of the interjoint members;
**characterised in that**:
extensible and contractible bottomed tubular resilient cover members (20a-20e) are fitted respectively over said finger mechanisms (1a-1e) including said damping members, said resilient cover members (20a-20e) having closed ends at tip ends of said finger mechanisms;
when curved portions of the resilient cover members which are produced when the finger mechanisms are bent enter gaps between adjacent ones of the damping members, front walls of the damping members (17,18,19,26,27) have a thickness for preventing the curved portions from being caught by the joints (9,10,11,23,24);
the resilient cover members (20a-20e) of the respective finger mechanisms are shaped complementarily to outer profiles of the finger mechanisms when the finger mechanisms are extended such that a restoring force is produced to bias the finger mechanisms to an extended position when the finger mechanisms are bent
the front walls of the resilient cover members (20a-20e) fitted over the finger mechanisms (1a-1e) have recesses (29a,29b,29c) defined therein at the respective joints (9,10,11,23,24) these recesses extending from the front wall toward side walls of the resilient cover members ; and
the thickness of portions of the resilient cover members (20a-20e) which are positioned at the recesses is smaller than the thickness of other portions of the resilient cover members.

2. A multi-fingered hand device according to claim 1, further **characterized in that** the gap between adjacent damping members (17, 18, 19, 26, 27) is shaped so as to spread away from the joints (9, 10, 11, 23, 24) and towards the front surface of the finger mechanisms (1a-1e).

3. A multi-fingered hand device according to claim 1 or 2, further **characterized in that** the resilient cover members (20a-20e) of the respective finger mechanisms (1a-1e) are thicker in at least a portion of back walls thereof behind the finger mechanisms than in front walls thereof.

4. A multi-fingered hand device according to any preceding claim, further **characterized in that** a resilient cover member removably mounted on said palm assembly (2) in covering relation to a surface of the palm assembly is integrally joined to at least one of said resilient cover members (20a-20e) of the respective finger mechanisms.

5. A multi-fingered hand device according to any one of claims 1 to 3, further **characterized in that** the resilient cover members (20a-20e) of the respective finger mechanisms (1a-1e) are made of an elastomer.

6. A multi-fingered hand device according to claim 4, further **characterized in that** the resilient cover members (20a-20e) of the respective finger mechanisms (1a-1e) and the resilient cover member of said palm assembly (2) are made of an elastomer.

7. A multi-fingered hand device according to claim 6, further **characterized in that** the recesses (29a,29b,29c) are spread toward the front walls of the resilient cover members (20a-20e).

## Patentansprüche

1. Vielfinger-Handvorrichtung aufweisend eine Mehrzahl von Fingermechanismen (1a-1e), die sich von einer Handflächenanordnung (2) aus erstrecken, wobei jeder der Fingermechanismen umfasst:
eine Mehrzahl von Zwischengelenkgliedern (6,7,8,21,22), die durch eine Mehrzahl von Fingergelenken (9,10,11,23,24) sukzessiv verbunden sind, wobei die Fingermechanismen (1a-1e) gebeugt und ausgestreckt werden können, wenn die Zwischengelenkglieder (6,7,8,21,22) um die Gelenke (9,10,11,23,24) gedreht werden;
und Dämpfungsglieder (17,18,19,26,27), die auf den jeweiligen Zwischengelenkgliedern (6,7,8,21,22) der Fingermechanismen in überdeckender Beziehung mit äußeren Umfangsflächen der Zwischengelerikglieder montiert sind, wobei die Dämpfungsglieder (17,18,19,26,27) jeweils auf Benachbarten der Zwischengelenkglieder (6,7,8,21,22) der Fingermechanismen (1a-1e) montiert sind, und voneinander an dem Gelenk (9,10,11,23,24) beabstandet sind, das zwischen den Benachbarten der Zwischengelenkglieder angebracht ist;
**dadurch gekennzeichnet,**
**dass** ausdehnbare und zusammenziehbare mit einem Boden versehene röhrenförmige spannkräftige Überdeckungsglieder (20a-20e) jeweils über den Fingermechanismen (1a-1e), einschließlich den Dämpfungsliedern angepasst sind, wobei die spannkräftigen Überdeckungsglieder. (20a-20e) geschlossene Enden an den Spitzen-Enden der Fingermechanismen aufweisen;
**dass**, wenn gekrümmte Abschnitte der spannkräftigen Überdeckungsglieder, die erzeugt werden wenn die Fingermechanismen gebeugt werden, in Spalten zwischen Benachbarten der Dämpfungsglieder eindringen, Vorderwände der Dämpfungsglieder (17,18,19,26,27) eine Dicke aufweisen, um die gekrümmten Abschnitte daran zu hindern, sich in den Gelenken (9,10,11,23,24) zu verfangen;
**dass** die spannkräftigen Überdeckungsglieder (20a-20e) der jeweiligen Fingermechanismen zu äußeren Profilen der Fingermechanismen, wenn die Fingermechanismen ausgestreckt sind, komplementär geformt sind, derart dass, wenn die Fingermechanismen gebeugt sind eine Rückstellkraft erzeugt wird um die Fingermechanismen zu einer ausgestreckten Stellung zurück zu spannen;
**dass** die Vorderwände der spannkräftigen Überdeckungsglieder (20a-20e), die über den Fingermechanismen (1a-1e) angepasst sind, Aussparungen (29a,29b,29c) aufweisen, die darin an den jeweiligen Gelenken (9,10,11,23,24) bestimmt sind, wobei diese Aussparungen sich von der Vorderwand aus zu Seitenwänden der spannkräftigen Überdeckungsglieder hin erstrecken; und
die Dicke von Abschnitten der spannkräftigen Überdeckungsglieder (20a-20e), die an den Aussparungen angebracht sind, kleiner ist als die Dicke von anderen Abschnitten der spannkräftigen Überdeckungsglieder.

2. Vielfinger-Handvorrichtung nach Anspruch 1, ferner **gekennzeichnet dadurch,**
**dass** der Spalt zwischen benachbarten Dämpfungsgliedern (17,18,19,26,27) derart geformt ist, um sich von den Gelenken (9,10,11,23,24) weg und zu der Vorderfläche der Fingermechanismen (1 a-1 e) hin auszubreiten.

3. Vielfinger-Handvorrichtung nach Anspruch 1 oder 2, ferner **gekennzeichnet dadurch,**
**dass** die spannkräftigen Überdeckungsglieder (20a-20e) der jeweiligen Fingermechanismen (1a-1e) dicker sind in mindestens einem Abschnitt von Rückwänden davon hinter den Fingermechanismen als in Vorderwände davon.

4. Vielfinger-Handvorrichtung nach einem vorhergehenden Anspruch, ferner **gekennzeichnet dadurch,**
**dass** ein spannkräftiges Überdeckungsglied, das abnehmbar auf der Handflächenanordnung (2) in überdeckender Beziehung mit einer Fläche der Handflächenanordnung montiert ist, integral mit mindestens einem der spannkräftigen Überdeckungsglieder (20a-20e) der jeweiligen Fingermechanismen verbunden ist.

5. Vielfinger-Handvorrichtung nach einem der Ansprüche 1 bis 3, ferner **gekennzeichnet dadurch,**
**dass** die spannkräftigen Überdeckungsglieder (20a-20e) der jeweiligen Fingermechanismen (1 a-1 e) aus einem Elastomer hergestellt sind.

6. Vielfinger-Handvorrichtung nach Anspruch 4, ferner **gekennzeichnet dadurch,**
**dass** die spannkräftigen Überdeckungsglieder (20a-20e) der jeweiligen Fingermechanismen (1a-1e) und das spannkräftige Überdeckungsglied der Handflächenanordnung (2) aus einem Elastomer hergestellt sind.

7. Vielfinger-Handvorrichtung nach Anspruch 6, ferner **gekennzeichnet dadurch,**
**dass** die Aussparungen (29a,29b,29c) zu den Vorderwänden der spannkräftigen Überdeckungsglieder (20a-20e) hin ausgebreitet sind.

## Revendications

1. Dispositif de main à multiples doigts comportant une pluralité de mécanismes de doigts (1a à 1e) s'étendant à partir d'un ensemble formant paume (2), comprenant chacun :
une pluralité d'éléments entre articulations (6, 7, 8, 21, 22) reliés successivement par une pluralité d'articulations de doigts (9, 10, 11, 23, 24), dans lequel lesdits mécanismes de doigts (1a à 1e) peuvent être pliés et étendus lorsque lesdits éléments entre articulations (6, 7, 8, 21, 22) sont tournés autour des articulations (9, 10, 11, 23, 24) ;
et des éléments d'amortissement (17, 18, 19, 26, 27) montés sur les éléments entre articulations (6, 7, 8, 21, 22) respectifs des mécanismes de doigts dans une relation de recouvrement des surfaces périphériques externes des éléments entre articulations, les éléments d'amortissement (17, 18, 19, 26, 27) étant respectivement montés sur les éléments adjacents parmi les éléments entre articulations (6, 7, 8, 21, 22) des mécanismes de doigts (1a à 1e) et étant espacés les uns des autres à l'articulation (9, 10, 11, 23, 24) disposée entre les éléments adjacents parmi les éléments entre articulations ;
**caractérisé en ce que** :
des éléments de recouvrement élastiques (20a à 20e) tubulaires munis de fonds, susceptibles d'extension et de contraction, sont respectivement montés sur lesdits mécanismes de doigts (1a à 1e) comprenant lesdits éléments d'amortissement, lesdits éléments de recouvrement élastiques (20a à 20e) comportant des extrémités fermées aux extrémités finales desdits mécanismes de doigts ;
lorsque des parties incurvées des éléments de recouvrement élastiques, qui sont produites lorsque les mécanismes de doigts sont pliés, pénètrent dans des espaces entre les éléments adjacents parmi les éléments d'amortissement, des parois avant des éléments d'amortissement (17, 18, 19, 26, 27) ont une épaisseur pour éviter que les parties incurvées soient pincées par les articulations (9, 10, 11, 23, 24) ;
les éléments de recouvrement élastiques (20a à 20e) des mécanismes de doigts respectifs sont formés de manière complémentaire aux profils externes des mécanismes de doigts lorsque les mécanismes de doigts sont étendus, de sorte qu'une force de rappel est produite pour solliciter les mécanismes de doigts vers une position étendue lorsque les mécanismes de doigts sont pliés ;
les parois avant des éléments de recouvrement élastiques (20a à 20e) montés sur les mécanismes de doigts (1a à 1e) comportent des évidements (29a, 29b, 29c) définis dans celles-ci aux articulations (9, 10, 11, 23, 24) respectives, ces évidements s'étendant de la paroi avant vers les parois latérales des éléments de recouvrement élastiques ; et
l'épaisseur des parties des éléments de recouvrement élastiques (20a à 20e) qui sont positionnées aux évidements est inférieure à l'épaisseur des autres parties des éléments de recouvrement élastiques.

2. Dispositif de main à multiples doigts selon la revendication 1, **caractérisé en outre en ce que** l'espace entre les éléments d'amortissement (17, 18, 19, 26, 27) adjacents est formé de manière à s'étendre à l'opposé des articulations (9, 10, 11, 23, 24) et vers la surface avant des mécanismes de doigts (1a à 1e).

3. Dispositif de main à multiples doigts selon la revendication 1 ou 2, **caractérisé en outre en ce que** les éléments de recouvrement élastiques (20a à 20e) des mécanismes de doigts (1a à 1e) respectifs sont plus épais dans au moins une partie des parois arrière de ceux-ci derrière les mécanismes de doigts que dans les parois avant de ceux-ci.

4. Dispositif de main à multiples doigts selon l'une quelconque des revendications précédentes, **caractérisé en outre en ce qu'**un élément de recouvrement élastique monté de manière amovible sur ledit ensemble formant paume (2) dans une relation de recouvrement avec une surface de l'ensemble formant paume est joint d'un seul tenant à au moins l'un desdits éléments de recouvrement élastiques (20a à 20e) des mécanismes de doigts respectifs.

5. Dispositif de main à multiples doigts selon l'une quelconque des revendications 1 à 3, **caractérisé en outre en ce que** les éléments de recouvrement élastiques (20a à 20e) des mécanismes de doigts (1a à 1e) respectifs sont réalisés en élastomère.

6. Dispositif de main à multiples doigts selon la revendication 4, **caractérisé en outre en ce que** les éléments de recouvrement élastiques (20a à 20e) des mécanismes de doigts (1a à 1e) respectifs et l'élément de recouvrement élastique dudit ensemble formant paume (2) sont réalisés en élastomère.

7. Dispositif de main à multiples doigts selon la revendication 6, **caractérisé en outre en ce que** les évidements (29a, 29b, 29c) sont étendus vers les parois avant des éléments de recouvrement élastiques (20a à 20e).
